(19) Europäisches Patentamt
European Patent Office
Office européen des brevets

(11) Numéro de publication: **0 255 715 B1**

# (12) FASCICULE DE BREVET EUROPEEN

(45) Date de publication de fascicule du brevet: **31.07.91**

(21) Numéro de dépôt: **87111247.0**

(22) Date de dépôt: **04.08.87**

(51) Int. Cl.5: **C07F 15/00**, C07D 221/14, C07D 217/26, C07D 225/06, C07D 471/04, C07D 471/06, C07D 335/06, C07D 337/16

## (54) Procédé de synthèse d'hétérocycles à partir de complexes palladocycliques.

(30) Priorité: **07.08.86 FR 8611455**
**08.01.87 FR 8700128**

(43) Date de publication de la demande:
**10.02.88 Bulletin 88/06**

(45) Mention de la délivrance du brevet:
**31.07.91 Bulletin 91/31**

(84) Etats contractants désignés:
**AT BE CH DE ES FR GB GR IT LI LU NL SE**

(56) Documents cités:

**JOURNAL OF THE CHEMICAL SOCIETY (C), 1970, pages 1764-1784; R.M. ACHESON et al.: "The synthesis, spectra, and reactions of some S-alkylthiophenium salts"**

**CHEMISTRY LETTERS, no. 3, mars 1986, pages 399-402, The Chemical Society of Japan; T. KITAMURA et al.: "Formation of 1-aryl-1-benzothiophenium ions in bromination of omicron-arylthiophenyl-substituted ethylenes"**

(73) Titulaire: **Europäische Wirtschaftsgemeinschaft Bâtiment Jean Monnet
Plateau du Kirchberg
L-2920 Luxemburg(LU)**

(72) Inventeur: **Pfeffer, Michel, Dr.
12 rue du Lièpvre
F-67100 Strasbourg(FR)**
Inventeur: **Maassarani, Figa
1 bd. de la Victoire
F-67000 Strasbourg(FR)**
Inventeur: **Dupont, Jairton
37, rue de la Curvau
F-67100 Strasbourg(FR)**

(74) Mandataire: **Weinmiller, Jürgen
Lennéstrasse 9 Postfach 24
W-8133 Feldafing(DE)**

**EP 0 255 715 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

JOURNAL OF THE AMERICAN CHEMICAL SOCIETY, vol. 96, no. 19, 18 septembre 1974, pages 6119-6132; A.G. HORTMANN et al.: "Thiabenzenes. IV. Synthesis and ylidic properties of 1-methyl-3,5-diphenylthiabenzene and 1-aryl-2-methyl-2-thianaphthalenes"

CHEMICAL ABSTRACTS, vol. 98, no. 21, 23 mai 1983, page 639, résumé no. 179167h, Columbus, Ohio, US; S. KLIMENKO et al.: "Synthesis and antimicrobial activity of S-R-sulfonium salts of bicyclic semidithioacetals", & KHIM.-FARM. ZH. 1983, 17(2),167-70

B. FRANCK et al.: "Liebigs Annalen der Chemie", 1983, no. 1, pages 425-432, Verlag-Chemie GmbH, Weinheim, DE; H. HOFMANN et al.: "Synthese und thermische Umlagerung von substituierten 1-methyl-4-phenyl-1-benzothiepinium-salzen-"

SYNTHESIS, no. 3, mars 1985, pages 233-252; A.D. RYABOV: "Cyclopalladated complexes in organic synthesis"

## Description

La présente invention concerne un procédé de synthèse d'hétérocycles à partir de complexes palladocycliques par reaction avec des alcynes, notamment un procédé de synthèse de composés hétérocycliques soufrés avec des rendements élevés, et les composés obtenus par ce procédé.

En vue d'effectuer des synthèses organiques, on a développé depuis plusieurs années des complexes organométalliques cyliques de métaux de transition dans lesquels la liaison σ métal carbone est stabilisée par une liaison de coordination intramoléculaire entre le métal et un atome donneur (N, P, O, S...)

Ces composés sont connus comme pouvant conduire à une réaction aisée avec divers réactifs en raison de la haute réactivité de la liaison métal-carbone.

Parmi ceux-ci on a développé en particulier des complexes du palladium II qui peuvent subir un grand nombre de réactions d'insertion A. Bahsoun et Coll (J. Chem. Soc Dalton Trans ,1979 ,547) ont ainsi décrit la réaction de complexes palladocycliques chlorés ou bromés avec les alcynes. Ils ont montré que suivant la nature de l'alcyne on obtient une mono- ou une di-insertion de l'alcyne dans la liaison Pd-C. En outre ils n'ont observé que dans un seul cas une démétallation spontanée conduisant à la formation d'un composé hétérocyclique.

On pourrait penser obtenir une voie générale de démétallation de tels composés organométalliques en faisant appel à des réducteurs classiques tels que les hydrures (Li Al $H_4$, NaH) mais l'utilisation de ces réducteurs risque d'induire ces réactions parasites (réduction delliaisons multiples par exemple).

La présente invention vise à fournir un procédé général pour obtenir une dépalladation effective lors de la réaction des complexes palladocycliques avec des alcynes, conduisant ainsi à la production d'hétérocycles par formation d'une liaison carbone-hétéroatome . Les inventeurs ont découvert qu'il était possible d'activer les complexes pallodocycliques en les mettant sous la forme de complexes iodés ou de complexes cationiques et d'obtenir lors de la réaction avec les alcynes une élimination du palladium par réduction de ce palladium à l'état métallique.

Ce but est atteint selon l'invention par le procédé tel qu'il est défini dans la revendication 1. Des exemples de mise en oeuvre préférée de ce procédé sont définis dans les revendications secondaires de procédé. Des composés obtenus par ce procédé sont définis dans les revendications de composés.

Les complexes palladocycliques que l'on utilise dans la présente invention peuvent être représentés par les formules schématiques suivantes :
complexe iodé :

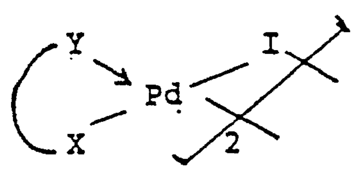

I

complexe cationique :

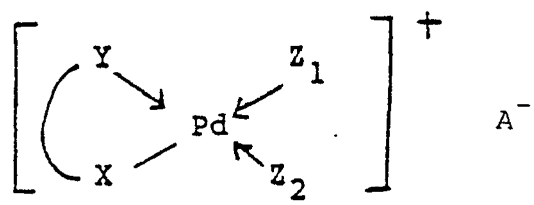

II

Dans ces formules Y - X représente un reste organique lié au palladium du côté Y par un atome d'azote ou de soufre et du côté X par un atome de carbone, $Z_1$ et $Z_2$ représentent deux ligands faiblement coordinés, et A représente un anion.

Dans ces complexes de formule I et II le palladium forme avec le reste Y - X un cycle ayant généralement de 5 à 7 chaînons (y compris le palladium). Comme exemple de restes Y - X présentant une liaison de coordination N→Pd on peut citer ceux dérivant des composés suivants :

- des alkylamines
- des benzylamines
- des arylamines

- des naphtylamines
- des imines
- des azobenzènes
- des bases de Schiff
- des hydrazones
- des pyroles
- des aminoferrocènes
- des oximes
- des pyridines
- des quinoléines
- des pyridines substituées

Des complexes azotés palladocycliques analogues sont décrits notamment dans l'article de A. Ryabov "Cyclopalladated Complexes in Organic Synthesis" Synthesis n° 3, p. 233, mars 1985.

Comme exemples de composés azotés formant de tels complexes on peut citer plus particulièrement :

- la 8-méthylquinoléine (8-mq)
- la N, N-diméthyl naphtylamine (dmna)
- la benzylpyridine (BP)
- la N,N-diméthylbenzylamine (dmba)

Comme exemples de restes Y - X présentant une liaison de coordination S → Pd on peut citer ceux dérivant des composés suivants :

- des arylthiocétones
- des benzylalkylthioéthers
- des naphthylalkylthioéthers
- des aryl alkyltioéthers
- des dérivés du thiophène tels que le 2-phényltiophène

Dans les complexes de formule II les ligands faiblement coordinés $Z_1$ et $Z_2$ peuvent être notamment des molécules de solvants faiblement coordinants tels que des nitriles RCN, des éthers R-O-R', des thioéthers R-S'-R', des cétones R-CO-R', des alcools ROH, le THF, le DMSO, le DMF (dans ces formules R et R' pouvant être des groupes alkyle en $C_1$ à $C_6$ ou aryle).

Les alcynes que l'on peut faire réagir selon l'invention avec les complexes iodés ou cationiques sont notamment des composés de formule

$R^1 - C \equiv C - R^2$ III

dans laquelle $R^1$ et $R^2$ sont choisis indépendamment l'un de l'autre parmi l'hydrogène, des groupes aryle tels que phényle, des groupes alkyle , notamment en $C_1$ à $C_6$, des groupes haloalkyle en $C_1$ à $C_6$; des groupes alcényle en $C_2$-$C_6$; des groupes de formule $-COOR_3$ ou $-CO-R_3$ dans lesquelles $R_3$ est un groupe alkyle en $C_1$ à $C_6$ ou aryle; des groupes de formule $-CR_4R_5$ OH dans laquelle $R_4$ et $R_5$ sont choisis parmi l'hydrogène, des groupes alkyle en $C_1$-$C_6$ et des groupes aryle; un groupe -CHO; des groupes de formule $-NR_6R_7$ dans laquelle $R_6$ et $R_7$ sont choisis parmi l'hydrogène et des groupes alkyle en $C_1$ à $C_6$; des groupes de formule $-SR_8$ ou $-SO_2R_8$ dans les lesquelles $R_8$ est choisi parmi des groupes alkyle en $C_1$ à $C_6$ et des groupes aryle; un groupe triméthylsilyle; ainsi que de tels groupes liés à la triple liaison par une chaîne alkylène en $C_1$ à $C_6$.

La présente invention en particulier concerne un procédé de synthèse d'hétérocycles soufrés de formule

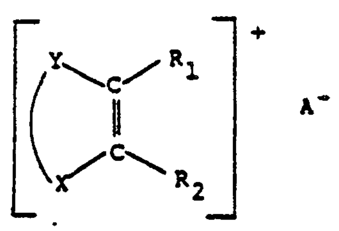

IV

dans laquelle -X-Y-représente un reste organique lié à la double liaison du côté Y par un atome de soufre chargé positivement et du côté X par un atome de carbone

$R_1$ et $R_2$ sont choisis indépendamment l'un de l'autre parmi l'hydrogène, des groupes aryle tels que

phényle, des groupes alkyle, notamment en $C_1$ à $C_6$, des groupes haloalkyle en $C_1$ à $C_6$; des groupes alcényle en $C_1$ à $C_6$; des groupes de formule $-COOR_3$ ou $-CO-R_3$ dans lesquelles $R_3$ est un groupe alkyle en $C_1$ à $C_6$ ou aryle; des groupes de formule $-CR_4R_5OH$ dans laquelle $R_4$ et $R_5$ sont choisis parmi l'hydrogène, des groupes alkyle en $C_1$ à $C_6$ et des groupes aryle; un groupe -CHO; des groupes de formule $-NR_6R_7$ dans laquelle $R_6$ et $R_7$ sont choisis parmi l'hydrogène et des groupes alkyle en $C_1$ à $C_6$; des groupes de formule $-SR_8$ ou $-SO_2R_8$ dans lesquelles $R_8$ est choisi parmi des groupes alkyle en $C_1$ à $C_6$ et des groupes aryle; un groupe triméthylsilyle; ainsi que de tels groupes liés à la double liaison par une chaîne alkylène en $C_1$ à $C_6$,

et A représente un anion, qui est caractérisé en ce que l'on fait réagir un complexe cationique de formule

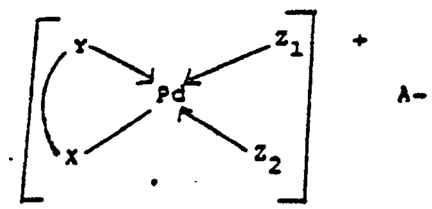

IIa

dans laquelle $Z_1$, $Z_2$, -X-Y- et A ont la signification donnée précédemment

avec un alcyne de formule (III) donnée précédemment

Ce procédé peut être appliqué notamment dans le cas où X - Y est un reste dérivant du sulfure de méthyle et de benzyle (par abbréviation mbS).

D'autres sulfures ou dérivés soufrés peuvent être utilisés et notamment

- des sulfures d'alkyle et aryle tels que des composés de formule

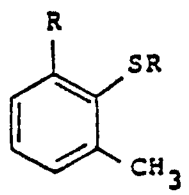

avec R = alkyle, aralkyle ou aryle

(IVa)

- des arylthiocétones tels que des composés de formule

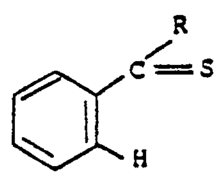

avec R = alkyle, aralkyle ou aryle

(IVb)

- des dérivés du thiophène tels que le 2-phénylthiophène.

Dans les complexes de formule II les ligands faiblement coordinants $Z_1$ et $Z_2$ peuvent être notamment des molécules de solvants faiblement coordinants comme definis ci-dessus.

Un ligand particulièrement approprié est l'acétonitrile (MeCN), c'est à dire qu'un complexe particulièrement approprié est un complexe cationique de formule

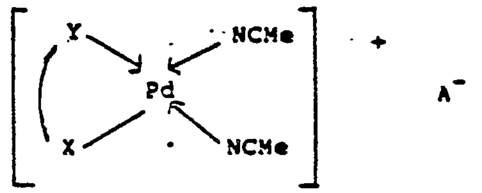

IIb

La réaction des complexes de formule I ou II avec les alcynes de formule III conduisant à une dépalladation du complexe et à la formation d'un hétérocycle peut généralement être effectuée par simple chauffage dans un solvant inerte tel que le chlorobenzène, le toluène ou le chloroforme, à la pression atmosphérique, généralement à une température de 125 à 135 °C, et notamment dans du chlorobenzène au reflux.

Le principal intérêt du procédé selon la présente invention est qu'il permet au départ d'amines tertiaires et d'alcynes substitués d'obtenir des hétérocycles fonctionnalisables en peu d'étapes. Le composé intermédiaire du palladium se fait généralement avec d'excellents rendements et il n'est généralement pas nécessaire de l'isoler. De plus ces composés sont facilement manipulables (leur synthèse peut se faire à l'air et à la température ambiante soit 20 à 25 °C) sans précaution particulière.

En ce qui concerne l'étape de la formation de l'hétérocycle elle se fait dans les conditions suffisamment douces de sorte qu'il n'y a pas de réaction parasite sur d'autres groupements fonctionnels présents sur l'amine métallée. Le procédé s'applique à toute une gamme d'amine. Le choix de celle-ci et du type de réaction utilisé permet d'obtenir des hétérocycles dont la taille varie généralement de 6 à 8 chainons.

La réaction est sélective. Dans la plupart des cas un seul produit est identifié après la réaction sans qu'il soit nécessaire d'utiliser des purifications supplémentaires. Lorsqu'un alcyne non symétrique est utilisé la réaction est régiosélective et souvent régiospécifique. La réaction est stoechiométrique en palladium mais celui-ci est évidemment récupérable et peut aisément être recyclé.

Le procédé peut être utilisé pour la synthèse d'hétérocycles difficilement accessibles par les méthodes de la synthèse organique classique. La structure des hétérocycles obtenus rappelle celle de certains alcaloïdes.

Ainsi le complexe iodé [{Pd (dmna) ( $\mu$- I) } $_2$] réagit avec des alcynes pour donner des hétérocycles selon le schéma réactionnel suivant :

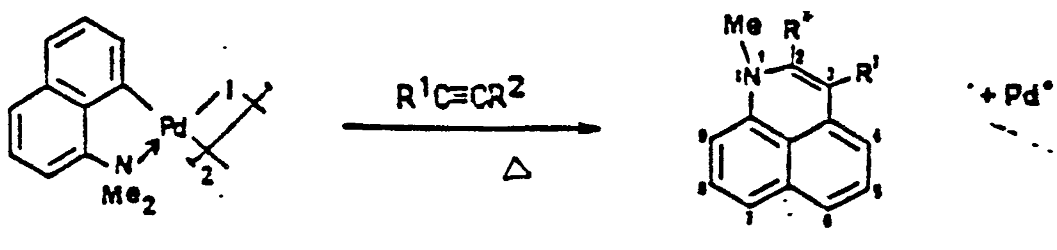


En revanche dans le cas du complexe chlore correspondant [{Pd (dmna) ($\mu$ - Cl)}$_2$ ] on n'observe de réaction qu'avec l'héxafluorobut-2 yne. Avec les autres alcynes on n'observe aucune réaction après chauffage au reflux dans du chlorobenzène pendant plusieurs jours.

Le complexe [Pd(dmna) (MeCN)$_2$] $BF_4$ réagit également avec des alcynes pour donner des hétérocycles selon le schéma suivant :

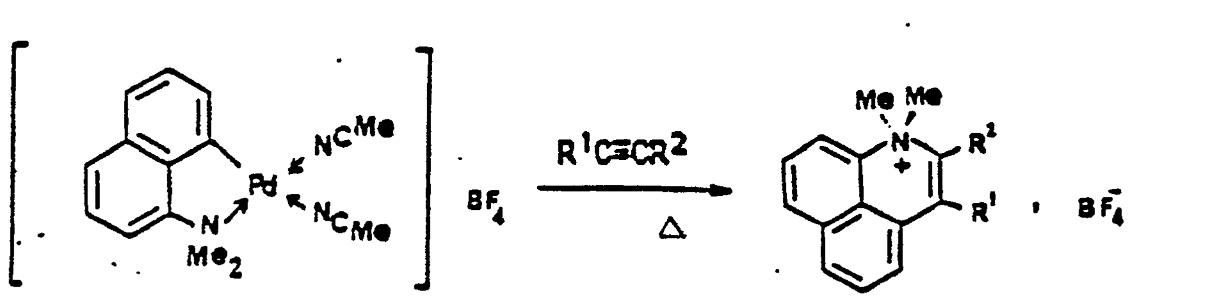


Les complexes cationiques de formule IIa peuvent être obtenus à partir des complexes halogénés

correspondants de formule

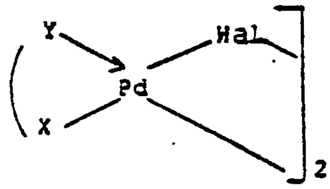

V

dans laquelle Hal représente un halogène notamment le chlore, le brome ou l'iode, avec un sel d'argent en présence d'un ou plusieurs ligands faiblement coordinants.

Comme complexe cationique de formule IIa on peut également utiliser des complexes de formule

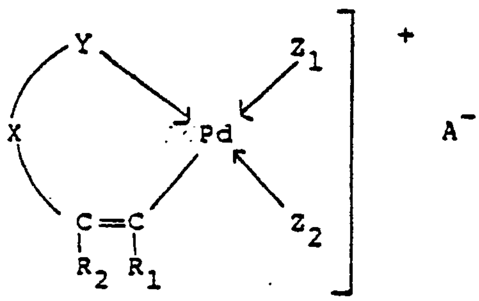

IIc

conduisant à des hétérocycles de formule

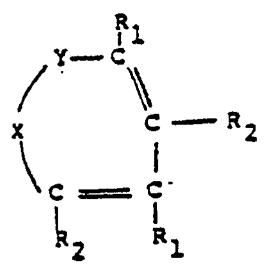

VI

Les complexes de formule IIc peuvent être obtenus à partir des complexes de formule V par réaction avec un alcyne de formule III conduisant à un complexe de formule

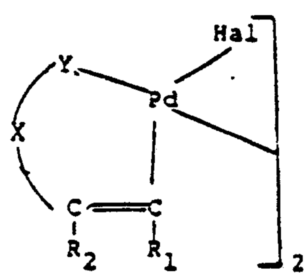

VII

et conversion de ce complexe halogéné en un complexe de formule IIc notamment par taitement par un sel d'argent en présence d'un solvant faiblement coordinant.

Les complexes halogénés de formule V peuvent être obtenus à partir d'un thioéther ou d'une thiocétone appropriés par réaction avec l'acétate de palladium puis réaction avec un halogénure de lithium.

On peut également effectuer des réactions d'échange de ligands comme illustré dans le schéma suivant

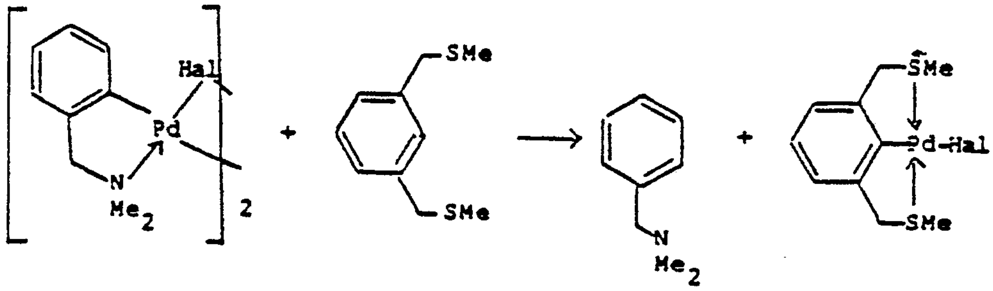

A la place des complexes halogénés de formule V on peut également utiliser des complexes halogénés de formule

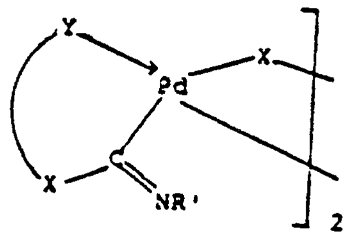

Va

qui sont obtenus à partir des complexes de formule V par réaction avec des isonitriles de formule R″NC dans laquelle R″ est notamment un groupe alkyle ou arylalkyle.

Ces composés Va peuvent être convertis en complexes cationiques et utilisés dans le procédé selon l'invention.

Le principal intérêt du procédé selon la présente invention est qu'il permet au départ de composés soufrés et d'alcynes substitués d'obtenir des hétérocycles fonctionnalisables en peu d'étapes. Le composé intermédiaire du palladium est obtenu généralement avec d'excellents rendements et il n'est généralement pas nécessaire de l'isoler. De plus ces composés sont facilement manipulables (leur synthèse peut se faire à l'air et à la température ambiante soit 20 à 25° C) sans précaution particulière.

Les exemples suivants illustrent la présente invention.

La partie A illustre la préparation des produits de départ et la partie B celle des hétérocycles. Dans la partie B les exemples 1 à 7 illustrent l'utilisation des complexes iodés et les exemples 8 à 16 illustrent l'utilisation des complexes cationiques.

A Préparation des produits de départ

1. Préparation des complexes iodés

a. Préparation du complexe[{ Pd (dmba) ( μ - I}₂]

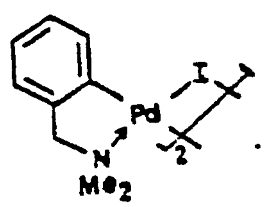

A une syspension du complexe pallodocyclique chloré [{ Pd (dmba) ( μ - Cl)}$_2$] correspondant (0,552 g, 1 mmole ) dans de l'acétone ( 50 ml ), on ajoute de l'iodure de sodium (0,6g,4 mmoles) . On obtient une solution fortement orangée. Après 5 minutes d'agitation on élimine le solvant sous vide et on extrait avec $CH_2$ $Cl_2$ ( 50 ml ) et on élimine $CH_2$ $Cl_2$ sous vide.

Spectre RMN$^1$H ($CDCl_3$)

3,99 et 3,96 (2s, 2H, $CH_2$-N) ; 2,91 et 2,88 (2s,6H, N-$CH_3$)

b. Préparation du complexe [{Pd (8-mq) ($\mu$-I)}$_2$]

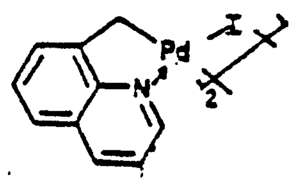

Il est obtenu comme sous 1 a à partir du complexe chloré correspondant.
RMN$^1$H ($CDCl_3$) : 9,29 (s(large), 1H, Ho) ; 8,25 (dd, 1H, Hp, $^3J$ Hp-Hm =7,4) ; 3,88 (s(large), 2H, $CH_2$).
c. Préparation du complexe [{Pd (dmna) ($\mu$-I)}$_2$]

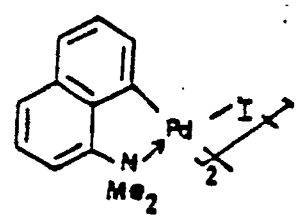

Il est obtenu comme sous 1 a à partir du complexe chloré correspondant
RMN$^1$ H (CDC$^1{}_3$) : 3,57 et 3,52 (2s, 6H, N-$CH_3$)

2. Préparation de complexes cationiques

a. Préparation du complexe de formule

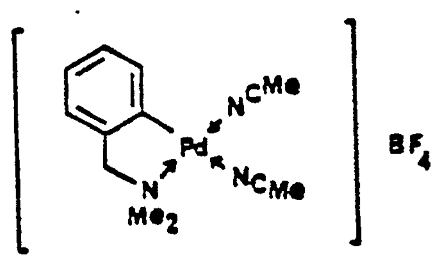

A une solution de Ag $BF_4$ (0,39g, 2 mmole) dans $CH_2$ $Cl_2$/Me CN (15 ml/1,5 ml) on ajoute une solution de[{ Pd (dmba) ($\mu$-Cl)}$_2$] (0,55g, 1 mmole) dans $CH_2$ $Cl_2$ (25ml), ce qui donne immédiatemment un précipité de AgCl. On filtre le mélange réactionnel sur une colonne de Celite (1 = 3cm). On concentre sous vide le filtrat incolore jusqu'à 2 ml, ce qui fournit par addition de pentane (30 ml ) du complexe cationique sous forme s'un solide blanc (0,65g, 80%).

I.R (nujol) :$\nu C \equiv N$ = 2332,2300(w)cm$^{-1}$.

RMN$^1$H ($CDCl_3$) : 3,91 (s, 2H, $CH_2$-N) ; 2,80 (s, 6H, N-$CH_3$) ; 2,47 et 2,28 (2s, 6H, $CH_3$-CN).

b. Préparation du complexe de formule

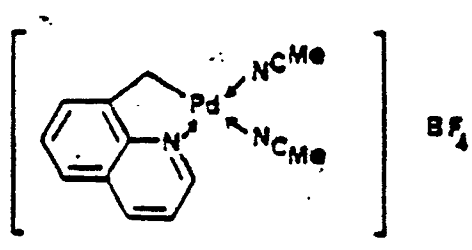

Il est préparé comme sous 2a à partir du complèxe chloré [{ Pd (8-mq) ($\mu$-Cl)}$_2$] (ce composé a été décrit par Brausstein et coll dans J Am Chem Soc, 1984, 106, 410)

c. Préparation du complexe de formule

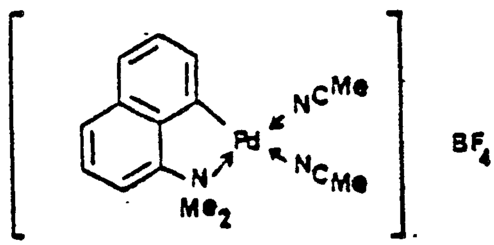

Il est préparé comme sous 2a à partir du complexe chloré [{Pd (dmna) ($\mu$-Cl}$_2$]
I.R (nujol) :$\nu$C≡N = 2310,2290(w) $cm^{-1}$
RMN $^1H$ ($CDCl_3$) : 3,36 (s,6H,N-$CH_3$) ; 2,55 et 2,33 (2s, 6H,$CH_3$-CN).
d. Préparation du complexe [Pd(mbS)($\mu$-Cl]$_2$

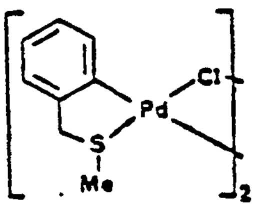

A une solution d'acétate de palladium (2,0 g, 9 mmole) dans de l'acide acétique glacial (125 ml) on ajoute du sulfure de méthyle et de benzyle (1,38 g, 10 mmole). Le mélange réactionnel est agité et chauffé sous reflux pendant 30 minutes. L'acide acétique est alors évaporé sous vide et le résidu noir obtenu est lavé avec du n-pentane (3x25 ml). A ce résidu, en suspension dans l'acétone (30 ml), on ajoute le chlorure de lithium en excès (2 g, 48 mmole), et ce mélange est agité pendant 10 minutes à la température ambiante. On évapore l'acétone sous vide, on place le résidu solide brun foncé sur une colonne de célite (25 mm de diamètre, 60 mm de hauteur) et le produit est extrait par du $CH_2Cl_2$(1l). La solution jaune orangée obtenue est concentrée sous vide (20 ml), on lui ajoute du pentane (100 ml) pour précipiter tout le produit sous forme d'une poudre jaune qui est lavée à l'eau (50 ml), séchée sous vide, relavée avec du pentane (50 ml) et reséchée sous vide. Rendement : 1,9 g, 76%. Spectre RMN $^1H$-($CDCl_3$) :
4,25 et 3,90 (2d,2H,$CH_2$,$CH_2$-S, $^2\tau_{HH}$ = 14,5 Hz), 2,63(s,-3H,$SCH_3$)
e) Préparation du complexe [Pd(mbS)(MeCN)-$_2$]$BF_4$

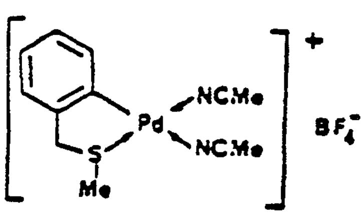

A une suspension de [Pd(mbS)($\mu$-Cl]$_2$(0,28 g, 0.5 mmole) dans $CH_2Cl_2$/MeCN(15 ml/1ml) on ajoute Ag $BF_4$ (0,195 g, 1 mmole). Au bout de 5 minutes d'agitation du mélange, on filtre le AgCl qui s'est formé sur une colonne de célite (1 = 3 cm) et la solution incolore obtenue est concentrée sous vide (2ml). L'addition de pentane (30 ml) fournit un précipité blanc qui est lavé à l'hexane (2x15 ml) est séché sous vide. Rendement : 340 mg, 81%.
IR(Nujol) : C = N 2295,2300 $cm^{-1}$
RMN$^1H$($CDCl_3$) : 4,21 et 3,83 (2d,2H,$CH_2$S, $^2\tau_{HH}$ = 14,5 Hz),2,63(s;3H,$SCH_3$),2,23(s(large),SH,$CH_3$CN).
f) Préparation du composé de formule

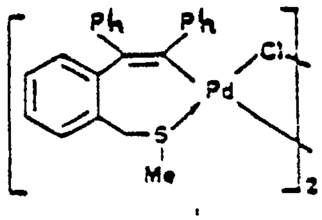

On fait réagir du diphénylacétylène (0,35 g, 1,96 mmole) avec $[Pd(mbS)(\mu\text{-}Cl)]_2$ (0,5 g, 0,9 mmole) dans le chlorobenzène (30 ml) au reflux pendant 30 minutes. Le solvant est éliminé sous vide et le résidu rouge est lavé avec du pentane (3x15 ml), puis dissous dans $CH_2Cl_2$ (5ml). Par l'addition de pentane (50ml) le produit précipite sous forme d'une poudre jaune. Rendement : 0,73 g, 80%.
RMN$^1$H($CDCl_3$) : 4,34 et 3,38 (2d,2H,$CH_2S$, $^1\tau_{HH}$ = 13.0 Hz),2,77 (s,3H,$SCH_3$).
g) Les composés correspondants où $R_1 = R_2 = CO_2.Me$ et où $R_1 = Ph, R_2 = CO_2Et$ ont été synthétisés de la même manière en utilisant le diméthylacétylène dicarboxylate et le 3-phénylpropiolate d'éthyle respectivement à la place du diphénylacétylène et en opérant à la température ambiante.

B. Préparation des hétérocycles

Exemple 1

Préparation du composé de formule

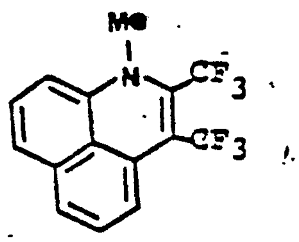

(composé 1)

On condense de l'héxafluorobut-2-yne (0,65g, 4 mmole) à la température de l'azote liquide dans un tube de Schlenk contenant une solution de $[\{Pd\ (dmna)\ (\mu\text{-}I)\}_2]$ obtenu sous 1 c (0,30g, 0,37 mmole) dans du chlorobenzène (25ml). On chauffe le mélange réactionnel à la température de reflux pendant 15h. On filtre la solution à travers une colonne de Celite (1 = 5cm) et on amène à sec sous vide. On obtient un solide vert. Une extraction avec le pentane (3x10 ml) fournit une solution orangé-rouge, qui après élimination du solvant donne le composé du titre à l'état pur sous la forme d'un solide rouge (0,12g, rendement 50%). Les caractéristiques du produit sont données dans le tableau I

Exemple 2

Préparation du composé de formule

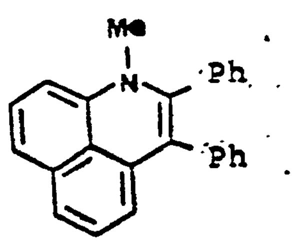

(composé 2)

On ajoute du diphénylacétylène (0,35g, 1,96 mmole) à une solution de $[\{Pd\ (dmna)\ (\mu\text{-}I)\}_2]$ (0,30g, 0,37 mmole) dans du chlorobenzène 40 ml). On chauffe le mélange au reflux pendant 24h et on filtre la solution noire obtenue sur une colonne de Celite (1 = 4cm) pour éliminer toute trace de palladium métallique. On amène la solution à siccité, ce qui fournit un produit brut consistant en le composé du titre et en

diphénylacétylène n'ayant pas réagi. On élimine ce dernier par chromatographie sur colonne d'alumine (1 = 12cm) en utilisant du pentane (230 ml) comme éluant. On extrait ensuite le produit pur avec de l'éther (40 ml) sous forme d'un solide jaune vert (0,12g, rendement 49 %) qui peut être cristallisé à - 20° C à partir d'une solution dans un mélange éther/pentane en donnant des cristaux jaunes fluorescents. F = 201° C. Les autres caractéristiques sont données dans le tableau I.

Exemple 3 Préparation du composé de formule

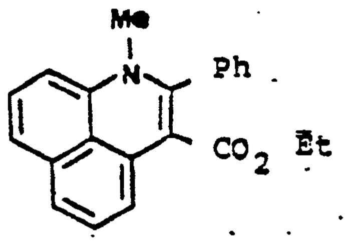

(composé 3)

On chauffe au reflux un mélange de [{Pd(dmna)(μ-I)}$_2$] (0,40g, 0,49 mmole) dans du chlorobenzène (45ml) avec du 3-phénylpropiolate d'éthyle (0,35g, 2 mmole) pendant 2 heures. On élimine le palladium métallique formé par filtration sur une colonne de Celite (1 = 4cm). On élimine le solvant sous vide et on lave le solide couleur kaki obtenu avec de l'hexane (5 ml) ce qui donne le composé du titre sous forme d'un solide vert (0,32g, rendement 96%) contaminé avec de l'alcyne libre. Une cristallisation dans une solution dans un mélange éther/pentane à -20° C donne des cristaux jaune-vert fluorescents du composé (0,13g, rendement 40%) F = 117° C. La liqueur mère est amenée à siccité et le résidu est purifié par chromatographie sur une colonne d'alumine (1 = 20cm). On extrait d'abord l'alcyne libre avec du pentane (500 ml). Une extraction avec de l'éther éthylique (250 ml) fourni le produit pur (0,09g, rendement de 30%) soit un rendement total de 70%. Les caractéristiques du composé sont données dans le tableau I.

Exemple 4

Préparation du composé de formule

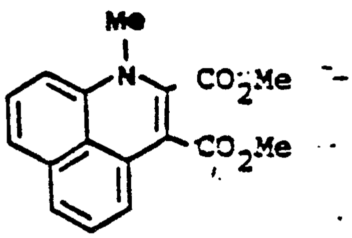

(composé 4)

On ajoute de l'acétylène dicarboxylate de diméthyle (0,20g, 1,4 mmole) à une solution de [{Pd (dmna)(μ-I }$_2$] (0,30g, 0,37 mmole) dans du chlorobenzène (40ml). Après agitation à la température de reflux pendant 70 minutes la couleur devient vert foncé. On filtre la solution à travers une colonne de Celite et on évapore le solvant sous vide. On lave le résidu vert avec de l'hexane (5 ml). l'extraction avec un mélange $CH_2Cl_2$/hexane (2 ml/15 ml) fournit une solution orangée qui après élimination du solvant donne le composé du titre sous forme de produit solide orangé (0,22g, rendement 50%). Lorsqu'on concentre la solution dans $CH_2Cl_2$/hexane et qu'on la refroidit à - 20° C on obtient le composé sous forme de cristaux orangés brillants (0,05g, rendement 23%) F = 92° C. D'autres caractéristiques sont données dans le tableau I

Exemple 5

Préparation du composé de formule

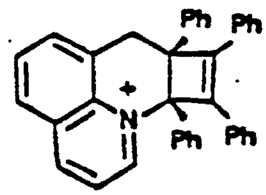 , $Pd\ I_3^-$ (composé 5)

## TABLEAU I

Caractéristiques des composés de formule générale 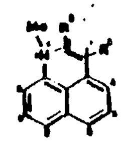

| Exemple | composé | couleur | I.R(KBr)$cm^{-1}$ $\nu$ C = O | $^1H$ RMN N-Me | $CO_2R$ | $H^9$ | $H^4$ |
|---|---|---|---|---|---|---|---|
| 1 | $R^1=R^2=CF_3$ | rouge | | 3,21(q) $^5J_{H-F}=2,2$ | | 6,35 $^3J_{H9-H8}=6,6$ | |
| 2 | $R^1=R^2=Ph$ | jaune vert | | 3,87(s) | | 6,23(d) $^3J_{H9-H8}=7,9$ | 6,13(d) $^3J_{H4-H5}=7,1$ |
| 3 | $R^1=CO_2Et$, $R^2=Ph$ | jaune vert | 1695(vs) | 3,86(s) | 3,80(q) 0,77(t) | 6,25(dd) $^3J_{H9-H8}=7,5$ | |
| 4 | $R^1=R^2=CO_2Me$ | orange | 1730.1690(vs) | 3,09(s) | 3,93(s) 3,81(s). | 6,30(dd) $^3J_{H9-H8}=6,9$ | |

On fait réagir du diphénylacétylène (0,27g, 1,52 mmole) avec[{Pd (8-mq) ($\mu$-I)}$_2$ ] (0,31g, 0,41 mmole) dans du chlorobenzène (50ml) au reflux pendant une demi-heure. On sépare par filtration le palladium métallique formé (0,04g, 63% par rapport à Pd) sur une colonne de Celite (1 = 4cm). On évapore sous vide la solution pourpre obtenue et on lave le résidu avec du pentane (3 x 10ml) et on dissout dans la quantité minimale de $CH_2Cl_2$. L'addition du pentane donne le composé du titre sous forme pure (0,24 g, rendement

88% par rapport à l'iode). On recristallise dans un mélange $CH_2$ $Cl_2$/pentane à température ambiante et l'on obtient des cristaux pourpres (0,16g, rendement 59%) .
RMN$^1$H : ($CDCl_3$) : 9,23 (dd, 1H, Ho, $^3\tau_{Ho\text{-}Hm}$ = 7,5), 8,65 (dd, 1H, Hp, $^3\tau_{Hp\text{-}Hm}$ = 6,0,$^4\tau_{Hp\text{-}Ho}$ = 0,8), 8,36 (dd, 1H, $H_m$) ; 5,14 et 4,09 (2d, 2H, $CH_2$,$^2\tau_{HA\text{-}HB}$ = 16,3) RMN $^{13}$C ($CDCl_3$) : 34,7 ($CH_2$).

Exemple 6

Préparation des composés de formule

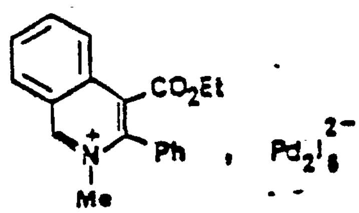

(6a)

et

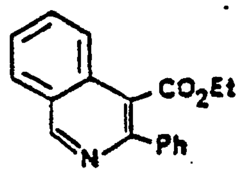

(6b)

a) Préparation du composé de formule

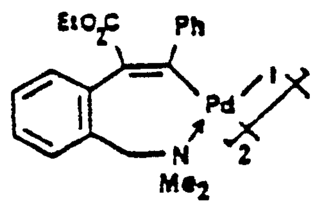

(composé 6')

On fait réagir du 3-phénylpropiolate d'éthyle (0,35 g, 2 mmoles) avec [{Pd(dmba)($\mu$-Cl)}$_2$] (0,55 g, 1 mmole) dans le dichlorométhane (50 ml) à la température ambiante pendant 1 heure. Le solvant est alors éliminé sous vide et le résidu est lavé avec du n-pentane (3 x 10 ml). On obtient un solide jaune qui est dissout dans $CH_2Cl_2$ (10 ml). On ajoute du pentane (15 ml) à cette solution et le mélange est refroidi à -20° C. Après 48 h, on obtient le composé [{Pd(dmba)C($CO_2$Et) = C(Ph)($\mu$-Cl)}$_2$](6") sous forme de cristaux jaune (0,53 g, 60%).

Le composé 6' est obtenu comme sous AAla sous forme d'un produit orange à partir du composé chloré 6".

RMN $^1$H($CDCl_3$ + ΣPy-d$^5$) = 4,13 et 3,04 (2d, 2H, $CH_2$-N, $^2\tau_{HH}$ = 10,7 Hz) 3,65 (1,2H,$CH_2$(Cet)), 3,10 et 2,06 (2S,6H,$CH_3$N), 0,61 (t,3H,$CH_3$(CEt)) $^3\tau_{HH}$=7,2 Hz).

b) Préparation des composés du titre

On chauffe au reflux pendant 3 heures une solution du produit obtenu en a (0,45g, 0,42 mmole) dans du chlorobenzène (50ml). On filtre la solution noire ainsi obtenue sur une colonne de Celite (1 = 4cm). On évapore le solvant sous pression réduite de la solution pourpre ainsi obtenue. On ajoute du pentane (35ml) et on agite pendant 3 heures. On filtre la solution obtenue pour séparer un solide foncé d'une solution jaune. Le solide est ensuite cristallisé à température ambiante dans un mélange $CH_2Cl_2$/pentane . On obtient le composé 6a sous forme de cristaux pourpres (0,01g rendement 14% par rapport à la dmba). L'évaporation du pentane sous vide conduit à une huile constituée par le composé 6b (0,10g, rendement 60% par rapport à la dmba).

Exemple 7

Préparation des composés de formule

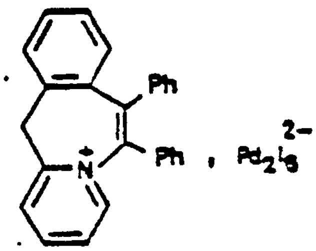

et

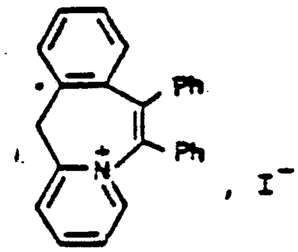

(7a) (7b)

On fait réagir un léger excès de diphénylacétylène (0,14g, 0,77 mmole) avec le complexe[{Pd (BP)(μ-I)}$_2$] (0,24g, 0,30 mmole) dans du chlorobenzène (40ml ) au reflux pendant 0,5 heure. On filtre la solution noire obtenue sur une colonne de Celite (1 = 4cm) pour éliminer le palladium métallique. On élimine ensuite le solvant et on lave le résidu pourpre avec du pentane (2 x 10ml). Le composé 7a cristallisé à partir d'une solution dans $CH_2$ $Cl_2$ (15 ml) sous forme de cristaux profondément pourpres (0,06g, rendement 36% par rapport à l'iode). On concentre la liqueur mère sous vide et le composé 7b précipite ensuite avec du pentane sous forme de solide orangé (0,08g, 43%). Le composé 7b peut être cristallisé dans un mélange $CH_2$ $Cl_2$/pentane (0,05g, 25%).

Composé 7a

RMN ($CD_3CN$) : 8,54 (dd, 1H, Ho,$^{3}\tau_{Ho\text{-}Hm}$ = 5,4), 8,42 (td, 1H, Hp,$^{3}\tau_{Hp\text{-}Hm}$ = 7,9,$^{4}\tau_{Hp\text{-}Ho}$ = 1,4), 8,17 (dd, 1H, Hm′, $^{3}\tau_{Hm\text{-}Hp}$ = 7,9,$^{4}{}_{Hm'\text{-}Hm}$ = 1,1), 4,70 et 4,59 (2d, 2H,$CH_2$, $^{2}\tau_{HA\text{-}HB}$ = 13,8) .

Composé 7b

RMN$^1$H ($CDCl_3$) : 8,81 (dd, 1H, Ho, $^{3}\tau_{Ho\text{-}Hm}$ = 6,6,$^{4}\tau_{Ho\text{-}Hp}$ = 1.1) 8,73 (dd, 1H, Hm′,$^{3}\tau_{Hm'\text{-}Hp}$ = 8,1,$^{4}\tau_{Hm'\text{-}Hm}$ = 1,2), 8,39 (td, 1H, Hp, $^{3}\tau_{Hp\text{-}Hm}$ = 7,8), 5,08 et 4,47 (2d,2H, $CH_2$,$^{2}{}_{HA\text{-}HB}$ =13,5)

Exemple 8

Préparation du composé de formule

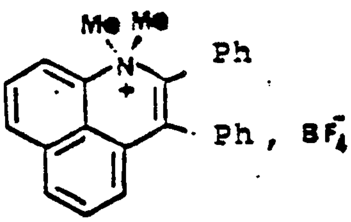

(composé 8)

On ajoute du diphénylacétylène (0,25g, 1,4 mmole) à une solution de [ Pd (dmna) (Me CN)$_2$ ]$BF_4$ (0,23g, 0,51 mmole) dans du chlorobenzène (60ml). On chauffe le mélange réactionnel au reflux pendant 1 heure, on filtre sur une colonne de Celite (1 = 5cm) pour éliminer le palladium métallique. On évapore sous vide la solution rouge foncé obtenue, ce qui fournit un solide qu'on lave avec du pentane (2 x 10ml). Le composé cristallise à partir d'un mélange $CH_2$ $Cl_2$ /pentane à température ambiante sous forme de cristaux pourpre foncé (0,04g, rendement 18%).

IR(KBr) : $\nu_{B\text{-}F}$ = 1050 (s,large) $cm^{-1}$

RMN$^1$H ($CD_3CN$) : 6,92 (d, 1H, $H^4$.3$\tau_{H4\text{-}H5}$ = 6,4), 3.76 (s, 6H,N-$CH_3$)

Exemple 9

Préparation du composé de formule

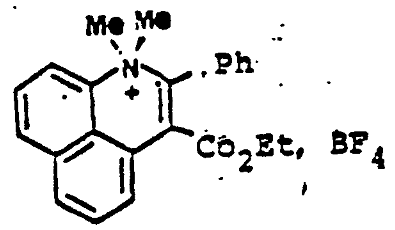

(composé 9)

On ajoute du 3-phénylpropiolate d'éthyle (0,15g, 0,86 mmole) à une solution de [Pd (dmna) $(MeCN)_2]BF_4$ - (0,20g, 0,46 mmole dans du chlorobenzène (50 ml). On chauffe le mélange réactionnel au reflux pendant deux heures, puis on élimine le palladium métallique par filtration sur une colonne de Celite (1 = 4cm). On obtient une solution vert foncé. On évapore sous vide le solvant et on lave le résidu ainsi obtenu avec du pentane (2 x 10ml) puis avec de l'éther (15ml) pour éliminer toutes traces de composé 3 formé lors de la réaction. Le solide vert restant consiste en le composé du titre ,contaminé avec [{Pd (dmna)($\mu$-Cl}$_2$] lié à l'utilisation du solvant chloré.
I.R (KBr) : $\nu_{C=O}$ = 1720(s) $cm^{-1}$ $\nu_{B-F}$ = 1050 (s, large)$cm^{-1}$
RMN'H ($CDCl_3$) : 4,02 (q,2H,O-$CH_2$), 3,89(s,6H,N-$CH_3$), 0,91 (t,3H,$CH_3$).

Exemple 10

Préparation du composé de formule

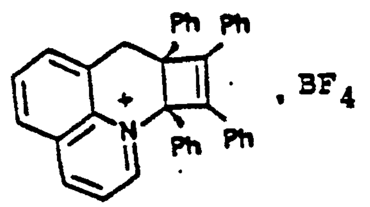

(composé 10)

On fait réagir du diphénylacétylène (0,30g, 1,68 mmole) avec le complexe [Pd(8-mq)(Me $CN)_2]BF_4$ (0,27g, 0,65 mmole) dans du chlorobenzène (50ml). On chauffe le mélange au reflux pendant 70 minutes et on filtre sur une colonne de Celite (1 = 5cm). On évapore le solvant sous vide et on lave le résidu avec du pentane (2 x 10ml). Le solide incolore obtenu est séché, ce qui fournit le composé du titre (0,32g, rendement 85%). On effectue ensuite une chromatographie sur une colonne d'alumine (1 = 6cm). L'élution . avec $CH_2$ $Cl_2$ - (20ml) élimine certaines impuretés. L'élution avec de l'acétone (40ml) fournit une solution incolore. On évapore sous vide l'acétone et on dissout le résidu dans la quantité minimale d'éther éthylique. Par refroidissement à - 20° C on obtient un solide blanc.
RMN'H : ($CDCl_3$) : 9,06 (dd,1H,Ho,$^3\tau_{Ho-Hm}$ = 7,2) : 8,69 (dd,1H,Hp,$^3\tau_{Hp-Hm}$ = 6,1) ; 8,24 (d(large),1H,Hm) ; 4,62 et 4,06 (2d,2H,$CH_2$,$^2\tau_{HA-HB}$ = 16,4).

Exemple 11

Préparation du composé de formule

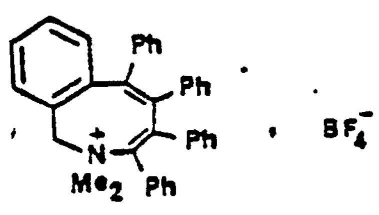

(composé 11)

A une solution de [ Pd(dmba)(Me $CN)_2]BF_4$ (0,25g, 0,61 mmole dans du chlorobenzène (50ml) on ajoute du diphénylacétylène (0,25g, 1,40 mmole) et on chauffe le mélange au reflux pendant 2,5 heures. On sépare par filtration sous une colonne de Celite (1 = 5cm) le palladium métallique ainsi formé. On évapore sous vide le solvant et on lave le résidu orangé avec du pentane (2 x 10 ml), on redissout avec la quantité

minimale de $CH_2$ $Cl_2$ et on précipite par addition de pentane (25 ml), ce qui fournit le composé du titre sous forme d'un solide orangé, avec un rendement quantitatif. Ce produit est purifié par cristallisation dans un mélange $CH_2$ $Cl_2$/pentane à -20° C ce qui fournit des cristaux jaune brillant (0,13g, rendement 37,5%) F = 204° C
IR (KBr) $\nu_{B-F}$ = 1070(s, large), $cm^{-1}$
RMN$^1$H ($CDCl_3$) : 5,83 (d,1H, proton aromatique, $^3\tau_{H-H}$ = 7,8), 4,21 et 3,88 (2d,2H,$CH_2$-N,$^2\tau_{HA-HB}$ = 13,6), 2,70 et 1,74 (2s (large), 6H,N-$CH_3$).

Exemple 12

Préparation du composé de formule

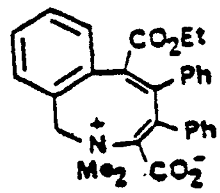

(composé 12)

On ajoute du 3-phénylpropiolate d'éthyle (0,26g, 1,49 mmole) à une solution de [Pd(dmba)$(MeCN)_2$] $BF_4$ - (0,25g, 0,61 mmole) Dans du chlorobenzène (40ml). On chauffe le mélange au reflux pendant 70 minutes. On obtient une solution noire. On élimine par filtration sur une colonne de Celite (1 = 4cm) le palladium métallique formé et l'on évapore le solvant sous vide. On purifie le produit solide résultant par chromatographie sous une colonne d'alumine (1 = 6cm) en utilisant $CH_2$ $Cl_2$ comme éluant. On évapore la solution sous vide et on obtient un produit solide cireux orangé.
IR (KBr) : $\nu_{C=O}$ = 1720(s) ($CO_2R$), 1615(m) ($CO_2^-$) $cm^{-1}$.
RMN$^1$ H ($CD_2$ $Cl_2$ ) : 7,70-6,94 (m, 14H, protons aromatique), 3,79(q,2H,O-$CH_2$), 3,22 et 3,33 (2d,2H,$CH_2$-N,$^2\tau_{HA-HB}$= 13, 8), 2,16 (s,6H,N-$CH_3$), 0,77(t,3H,$CH_3$).

Exemple 13

Préparation du composé de formule.

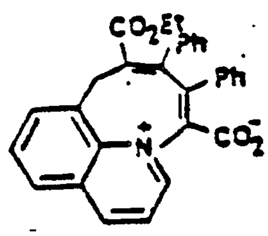

(composé 13)

On opère comme à l'exemple 12 à partir de [Pd(8-mq)$(MeCN)_4$] $BF_4$ (0,21g, 0,5 mmole) et de 3-phénylpropiolate d'éthyle (0,21g, 1,2 mmole) dans du chlorobenzène (50ml)
IR (KBr) : $\nu_{C=O}$ = 1715(s)($CO_2R$),1615(m) ($CO_2^{-1}$ )
RMN$^1$H($CD_2Cl_2$) : 8,80 (dd,1H,Ho,$^3\tau_{Ho-Hm}$= 4.2,$^4\tau_{Ho-Hp}$ = 1,8), 8,14 (dd, 1H,Hp,$^3\tau_{Hp-Hm}$ = 8,3), 7,70-7,17 (m, 14H, protons aromatique), 4,38 (s(large), 2H, $CH_2$), 3,79(q,2H,O-$CH_2$), 0,80(t,3H,$CH_3$).

Exemple 14

Préparation du composé de formule

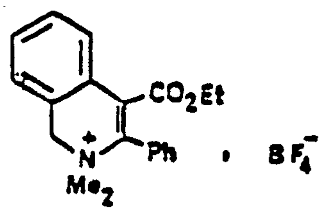

(composé 14)

a) préparation du composé de formule

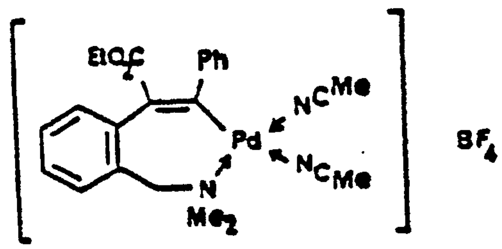

(composé 14')

Le composé 14' est obtenu comme sous A 2a à partir du composé chloré 6''.
RMN$^1$H($CDCl_3$):3,95 et 3,22(2d, 2H, $CH_2$-$\overline{N}$,$^2\tau_{HH}$ = 13,5 Hz), 3,84(q,2H, $CH_2$(OEt)), 2,80 et 2,57 (2s, 6H, $CH_3$-N), 2,16 et 2,03 (2s, 6H, $CH_3$-CN), 0.77 (t, 3H, $CH_3$(OEt), $^3\tau_{HH}$ = 7,1 Hz).

b) préparation du composé du titre.

On chauffe le composé obtenu en a (0,65g, 1,n mmole) dans du chlorobenzène (40ml) au reflux pendant 0,75 heures. On obtient une formation quantitative de palladium métallique. On filtre le mélange réactionnel sur une colonne de Celite (1 = 4cm) et on évapore le solvant sous vide. Le résidu ainsi obtenu est purifié par chromatographie sur colonne d'alumine (1 = 7cm). On utilise $CH_2$ $Cl_2$ (50 ml) come éluant pour éliminer les impuretés puis on extrait avec de l'acétone (35ml). Après élimination du solvant on obtient un solide incolore (0,18g, 41%).
I.R (KBr) : $\nu_{C=O}$ = 1725(s) ($CO_2R$) $cm^{-1}$ ; $_{B-F}$ = 1060 (s, large) $cm^{-1}$
RMN$^1$ H($CDCl_3$) : 5,06 (s,2H,$CH_2$-N), 3,99(q,2H,O-$CH_2$), 3,26 (s,6H,N-$CH_3$), 0,85(t,3H,$CH_3$).

EXEMPLE 15

Préparation du composé de formule

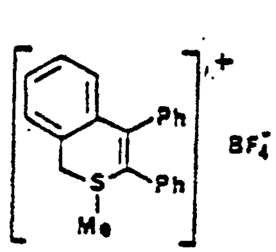

(composé 15)

On ajoute du diphénylacétylène (0,234 g, 1,3 mmole) à une suspension de /Pd(mbS)$(MeCN)_2$/$BF_4$(0,26 g, 0,61 mmole) dans du chlorobenzène (30 ml). On chauffe le mélange au reflux pendant 15 minutes et on filtre la solution noire obtenue sur une colonne de Célite (1 = 4cm) pour éliminer toute trace de palladium métallique. Le chlorobenzène de la solution rouge est éliminé sous vide et le résidu est dissous dans $CH_2Cl_2$ (3 ml). Le produit est précipité par addition de pentane (15 ml) sous forme d'une poudre jaune qui est lavée au pentane (3x15 ml) et séchée sous vide. Rendement 0,175 g, 90%. le produit est recristallisé dans une solution $CH_2Cl_2$/pentane(5 ml/20 ml) (rendement : 110 mg, 63%). RMN$^1$H($CDCl_3$):5,14 et 5,07-(2d,2H,$CH_2$S, $^2\tau_{HH}$ = 16,1 Hz), 2,97 (s,3H,$SCH_3$).

EXEMPLE 16

Préparation du composé de formule

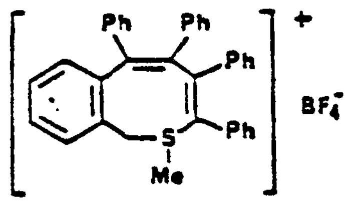

BF$_4^-$ (composé 16)

a) Première méthode

On fait réagir du diphénylacétylène (0,23 g, 1,27 mmole) avec [Pd(mbS)($\mu$-Cl)]$_2$ (0,17 g, 0,3 mmole) dans le toluène (50 ml) au reflux pendant 45 mn. La solution orange rouge est concentrée sous vide (4 ml) et on lui ajoute du pentane (75 ml). On obtient un précipité jaune orange (0,3 g) qui est lavé au pentane et séché sous vide. Ce composé est mis en solution dans $CH_2Cl_2$/MeCN (15 ml/1 ml). L'addition de $AgBF_4$ (0,05 g, 0,25 mmole) provoque l'apparition d'AgCl qui est filtré au bout de 5 mn et le solvant est éliminé sous vide. On obtient une huile jaune qui est dissoute dans le toluène (50 ml). Cette solution est chauffée au reflux pendant 20 mn. Le palladium métallique formé est filtré sur une colonne de Célite (1 = 3 cm). La solution orange ainsi obtenue est concentrée sous vide (3 ml). L'addition d'hexane (25 ml) permet d'isoler le composé attendu sous la forme d'une poudre orange qui est lavé à l'hexane (20 ml) et séché sous vide. Rendement 0,19 g, 83%.

b) Deuxième méthode

On fait réagir le composé obtenu sous A-2d (0,25 g, 0,28 mmole) en solution dans $CH_2Cl_2$/MeCN (15 ml) avec $AgBF_4$ (0,11 g, 0,55 mmole). On filtre le AgCl formé et on évapore le solvant sous vide pour obtenir une huile jaune. On ajoute à une solution de celle-ci dans le chlorobenzène (50 ml) du diphényl acétylène (0,1 g, 0,56 mmole) et on chauffe le mélange réactionnel au reflux pendant 1 heure. Le palladium métallique est filtré sur Célite et l'on isole le produit désiré comme ci-dessus avec un rendement similaire.

RMN$^1$H($CD_2Cl_2$,-80 °C) : 4,99 et 4,81 (2 d,2H,$CH_2S$, $^2\tau_{HH}$ = 16,4 Hz), 2,89 (s,3H,$SCH_3$).

**Revendications**

1. Procéde de synthèse d'hétérocycles à partir de complexes palladocycliques, par réaction avec des alcynes, caractérisé en ce que l'on utilise les complexes palladocycliques sous la forme d'un complexe iodé de formule

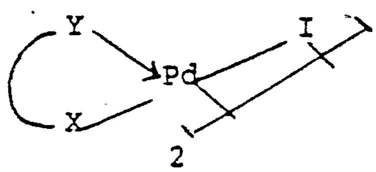

(I)

ou sous la forme d'un complexe cationique de formule

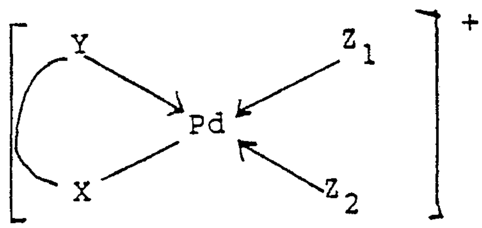

A$^-$ (II)

dans laquelle Y - X représente un reste organique lié au palladium du côté Y par un atome d'azote ou de soufre et du côté X par un atome de carbone, $Z_1$ et $Z_2$ représentent deux ligands faiblement

coordinés, tels que des nitriles RCN, des éthers RD-R', des thioéthers R-S'-R', des cétones R-CO-R', des alcools ROH, le THF, le DMSO, le DMF (dans ces formules, R et R' peuvent être des groupes alkyle en $C_1$ à $C_6$ ou aryle), et A représente un anion.

**2.** Procédé selon la revendication 1, caractérisé en ce que Y - X est un reste dérivant de la 8 - méthylquinoléine, la N,N - diméthyl naphtylamine, la benzylpyridine et la N,N - diméthylbenzylamine.

**3.** Procédé selon l'une quelconque des revendications 1 à 2, caractérisé en ce que on utilise un alcyne de formule

$R^1 - C \equiv C - R^2$ (III)

dans laquelle $R^1$ et $R^2$ sont choisis indépendamment l'un de l'autre parmi l'hydrogène, des groupes aryle tels que phényle, des groupes alkyle, notamment en $C_1$ à $C_6$, des groupes haloalkyle en $C_1$ à $C_6$; des groupes alcényle en $C_2$-$C_6$; des groupes de formule $-COOR_3$ ou $-CO-R_3$ dans lesquelles $R_3$ est un groupe alkyle en $C_1$ à $C_6$ ou aryle; des groupes de formule $-CR_4R_5$ OH dans laquelle $R_4$ et $R_5$ sont choisis parmi l'hydrogène, des groupes alkyle en $C_1$-$C_6$ et des groupes aryle; un groupe -CHO; des groupes de formule $-NR_6R_7$ dans laquelle $R_6$ et $R_7$ sont choisis parmi l'hydrogène et des groupes alkyle en $C_1$ à $C_6$; des groupes de formule $-SR_8$ ou $-SO_2R_8$ dans les lesquelles $R_8$ est choisi parmi des groupes alkyle en $C_1$ à $c_6$ et des groupes aryle; un groupe triméthylsilyle; ainsi que de tels groupes liés à la triple liaison par une chaîne alkylène en $C_1$ à $C_6$.

**4.** Procédé selon l'une quelconque des revendications 1 à 3, caractérisé en ce que l'on effectue la réaction par chauffage dans un solvant inerte.

**5.** Procédé selon la revendication 3, caractérisé en ce que l'on fait réagir un complexe iodé de formule

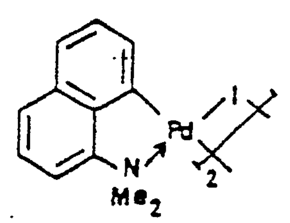

avec un alcyne de formule

$R^1 - C \equiv C - R^2$

$R^1$ et $R^2$ ayant la signification donnée à la revendication 3, et l'on obtient un hétérocycle de formule

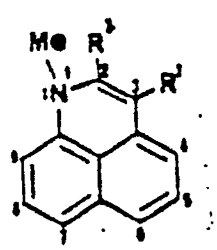

$R^1$ et $R^2$ ayant la signification donnée précédemment.

**6.** Procédé selon la revendication 3, caractérisé en ce que l'on fait réagir un complexe cationique de formule

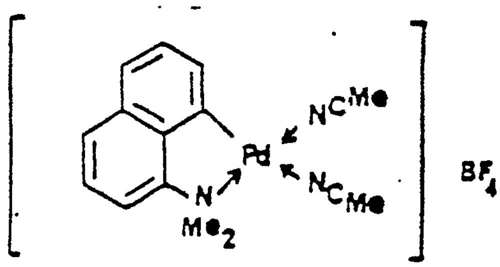

avec un alcyne de formule

$R^1 - C \equiv C - R^2$

$R^1$ et $R^2$ ayant la signification donnée a la revendication 3 et l'on obtient un hétérocycle de formule

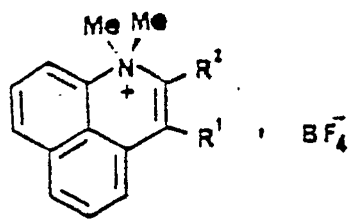

dans laquelle $R^1$ et $R^2$ ont la signification donnée précédemment.

**7.** Procédé de synthèse d'hétérocycles soufrés de formule

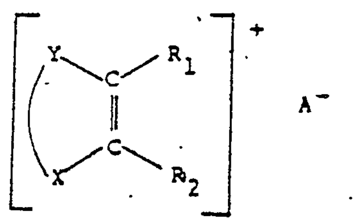

(IV)

dans laquelle -X-Y- représente un reste organique lié à la double liaison du côté Y par un atome de soufre chargé positivement et du côté X par un atome de carbone

$R_1$ et $R_2$ sont choisis indépendamment l'un de l'autre parmi l'hydrogène, des groupes aryle, des groupes alkyle, notamment en $C_1$ à $C_6$, des groupes haloalkyle en $c_1$ à $C_6$; des groupes alcényle en $C_1$ à $C_6$; des groupes de formule $-COOR_3$ ou $-CO-R_3$ dans lesquelles $R_3$ est un groupe alkyle en $C_1$ à $C_6$ ou aryle; des groupes de formule $-CR_4R_5OH$ dans laquelle $R_4$ et $R_5$ sont choisis parmi l'hydrogène, des groupes alkyle en $c_1$ à $c_6$ et des groupes aryle; un groupe -CHO; des groupes de formule $-NR_6R_7$ dans laquelle $R_6$ et $R_7$ sont choisis parmi l'hydrogène et des groupes alkyle en $C_1$ à $C_6$; des groupes de formule $-SR_8$ ou $-SO_2R_8$ dans lesquelles $R_8$ est choisi parmi des groupes alkyle en $C_1$ à $C_6$ et des groupes aryle; un groupe triméthylsilyle; ainsi que de tels groupes liés à la double liaison par une chaine alkylène en $C_1$ à $C_6$, et

A représente un anion,

selon l'une quelconque des revendications 1 à 4, characterisé en ce que l'on fait réagir un complexe cationique de formule

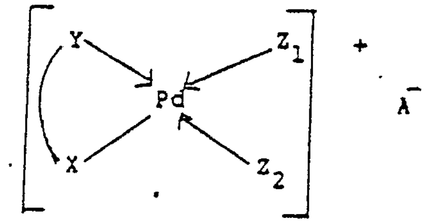

(IIa)

dans laquelle -X-Y- et A ont la signification donnée précédemment et $Z_1$ et $Z_2$ représentent deux ligands faiblement coordinants avec un alcyne de formule

$R_1 - C \equiv C - R_2$ (III)

**8.** Procédé selon la revendication 7, caractérisé en ce que l'on utilise un complexe cationique de formule

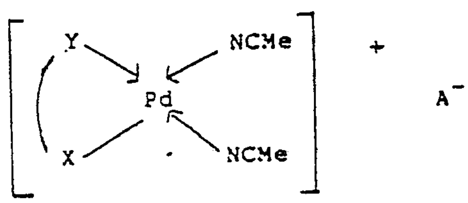

( IIb )

**9.** Procédé selon la revendication 7, caractérisé en ce que l'on prépare les complexes cationiques de formule (IIa) par réaction d'un complexe de formule

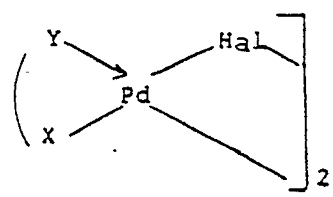

dans laquelle Hal représente un halogène avec un sel d'argent en présence d'un ou plusieurs ligands faiblement coordinants.

**10.** Procédé selon la revendication 7, caractérisé en ce que l'on utilise un complexe de formule

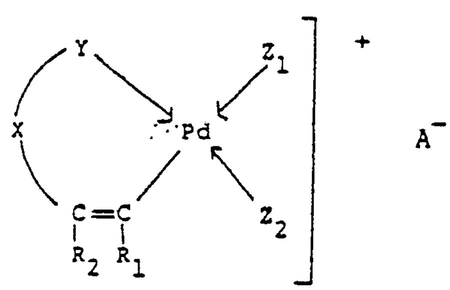

( IIc )

et l'on obtient un composé hétérocylique de formule

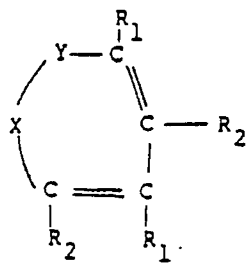

( VI )

**11.** Procédé selon la revendication 10, caractérisé en ce que l'on prépare le complexe de formule (IIc) à partir d'un complexe de formule

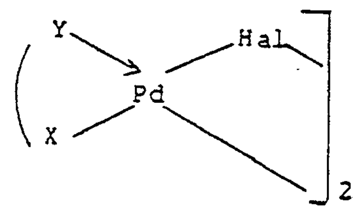

( V )

par réaction avec un alcyne de formule

$R_1 - C \equiv C - R_2$ (III)

conduisant à un complexe de formule

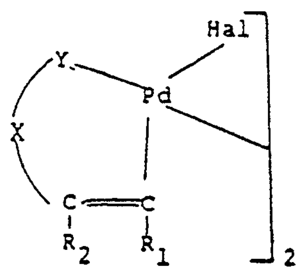

( VII )

et l' on convertit ce complexe en un complexe de formule IIc.

**12.** Composé hétérocyclique soufré de formule

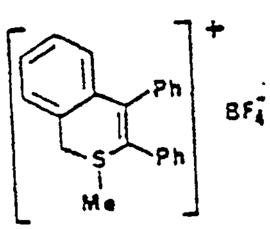

**13.** Composé hétérocyclique de formule

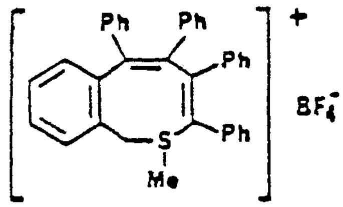

**14.** Composé intermédiaire de formule

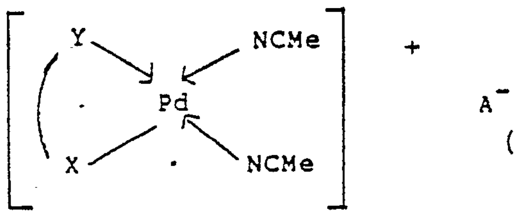

(IIb)

dans lequelle -X-Y- représente un reste organique lié à la double liaison du côté Y par un atome de soufre et au côté X par un atome de carbone, et
A représente un anion.

**15.** Composé selon la revendication 14 de formule

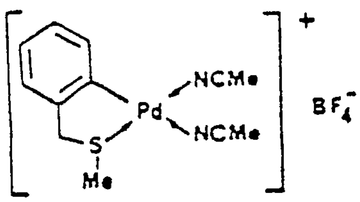

**16.** Complexes halogénés de formule

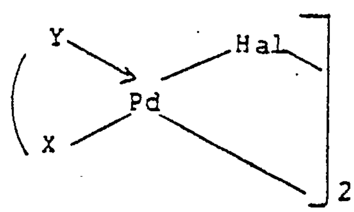

(V)

dans laquelle Hal représente un halogène et -X-Y- représente un reste organique lié à la double liaison du côté Y par un atome de soufre et du côté X par un atome de carbone.

**17.** Composés selon la revendication 16 de formule

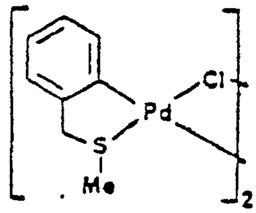

**18.** Complexes cationiques de formule

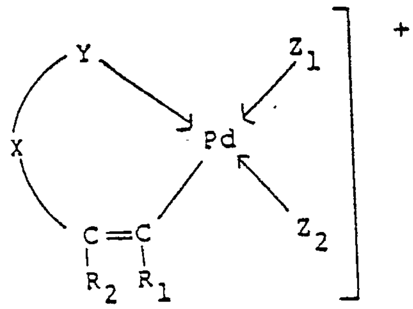

( IIc)

dans laquelle -X-Y-, $R_1$, $R_2$, $Z_1$ et $Z_2$ ont la signification donnée à la revendication 7.

**19.** Composés halogénés de formule

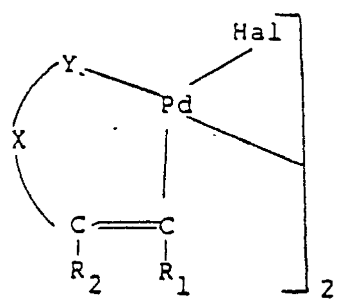

( VII )

dans laquelle -X-Y-, $R_1$, $R_2$ ont la signification donnée à la revendication 7.

**20.** Composés selon la revendication 19 de formule

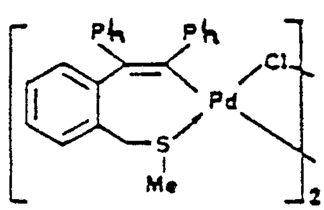

## Claims

**1.** A process of synthetisizing heterocycles starting from cyclo-palladium complexes, by the reaction with alkynes, characterized in that cyclo-palladium complexes are used in the form of iodine containing complexes of the formula:

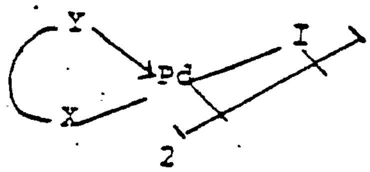

(I)

or cationic complexes of the formula:

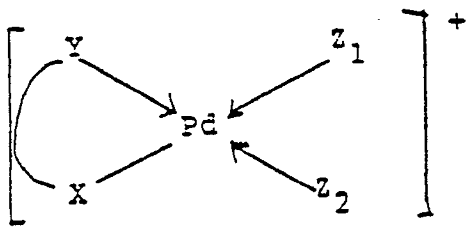

A$^-$ (II)

wherein Y -X represents an organic residue bonded to palladium at Y via a nitrogen atom or a sulphur atom, and at X via a carbon atom; $Z_1$ and $Z_2$ represent two weakly coordinated ligands; nitriles RCN, ethers R-O-R',thioethers R-S'-R', ketones R-CO-R', alcohols RON, THF, DMSO, DMF (in these formulas, R and R' be $C_1$-$C_6$-alkyl groups or aryl groups), and A represents an anion.

2. The process according to claim 1, characterized in that Y-X is a residue derived from 8-methyl-chinolein, N,N-dimethylnaphtylamine, benzylpyridine and N,N-dimethylbenzolamine.

3. The process according to any one of claims 1 and 2, characterized in that an alkyne is used having the formula:

$$R^1 - C \equiv C - R^2 \qquad (III)$$

wherein $R^1$ and $R^2$ are selected independently from the group consisting of hydrogen, aryl groups such as phenyl, alkyl groups, especially $C_1$-$C_6$-alkyl groups, halo-$C_1$-$C_6$-alkyl groups; $C_2$-$C_6$-alkenyl groups; groups of the formula $-COOR_3$ or $-CO-R_3$, in which $R_3$ is a $C_1$-$C_6$-alkyl group or an aryl group; groups of the formula $-CR_4R_5OH$, in which $R_4$ and $R_5$ are selected from the group consisting of hydrogen, $C_1$-$C_6$-alkyl groups and aryl groups; a group -CHO, groups of the formula $-N_6R_7$, in which $R_6$ and $R_7$ are selected from hydrogen and $C_1$-$C_6$-alkyl groups; groups of the formula $-SR_8$ or $-SO_2R_8$, in which $R_8$ is selected from $C_1$-$C_6$-alkyl groups and aryl groups; a trimethylsilyl group; as well as such groups linked to a triple bond via a $C_1$-$C_6$-alkylene chain.

4. The process according to any one of claims 1 to 3, characterized in that the reaction is carried out by heating in an inert solvent.

5. The process according to claim 3, characterized in that a iodine containing complex of the formula

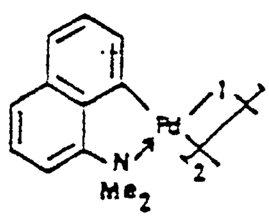

is reacted with an alkyne of the formula

$$R^1 - C \equiv C - R^2$$

wherein $R^1$ and $R^2$ have the meaning given in claim 3, so as to obtain a heterocycle of the formula:

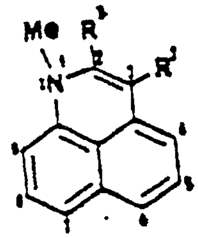

wherein $R^1$ and $R^2$ have the meaning given before.

**6.** The process according to claim 3, characterized in that a cationic complex of the formula

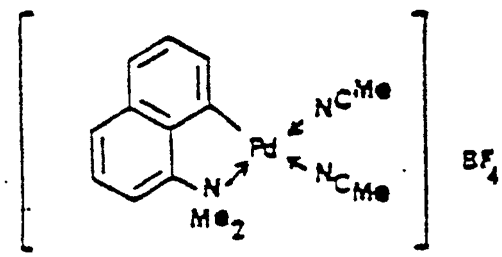

is reacted with an alkyne of the formula

$R^1 - C \equiv C - R^2$

wherein $R^1$ and $R^2$ have the meaning given in claim 3, so as to obtain a heterocycle of the formula:

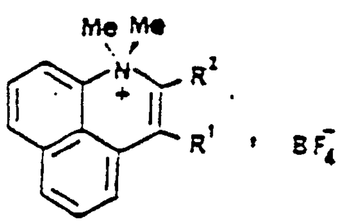

wherein $R^1$ and $R^2$ have the meaning given before.

**7.** The process of synthesizing sulphur containing heterocycles of the formula:

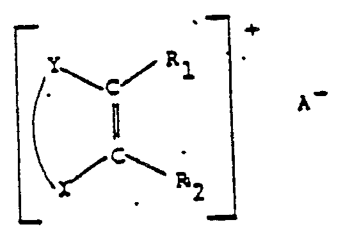

(IV)

wherein -X-Y- represents an organic residue linked to the double bond at Y via a positively charged sulphur atom, and at X via a carbon atom; wherein $R^1$ and $R^2$ are selected independently from the group consisting of hydrogen, aryl groups such as phenyl, alkyl groups, especially $C_1$-$C_6$-alkyl groups, halo$C_1$-$C_6$-alkyl groups; $C_2$-$C_6$-alkenyl groups; groups of the formula $-COOR_3$ or $-CO-R_3$, in which $R_3$ is a $C_1$-$C_6$-alkyl group or an aryl group; groups of the formula $-CR_4R_5OH$, in which $R_4$ and $R_5$ are selected from hydrogen, $C_1$-$C_6$-alkyl groups and aryl groups; a group -CHO, groups of the formula

$-NR_6R_7$, in which $R_6$ and $R_7$ are selected from hydrogen and $C_1$-$C_6$-alkyl groups; groups of the formula $-SR_8$ or $-SO_2R_8$, in which $R_8$ is selected from $C_1$-$C_6$-alkyl groups and aryl groups; a trimethylsilyl group; as well as such groups linked to a triple bond via a $C_1$-$C_6$-alkylene chain, and A represents an anion,
according to any one of claims 1 to 4, characterized in that a cationic complex of the formula

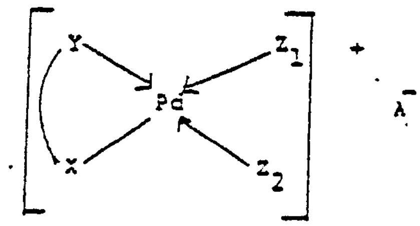
(IIa)

wherein -X-Y- and A have the meaning given above and $Z_1$ and $Z_2$ represent two weakly coordinating ligands, are reacted with an alkyne of the formula

$R_1 - C \equiv C - R_2$ (III)

8. The process according to claim 7, characterized in that a cationic complex is used which has the following formula:

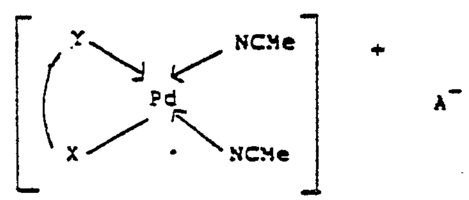
(IIb)

9. The process according to claim 7, characterized in that the cationic complexes having the formula (IIa) are obtained by reacting a complex of the formula:

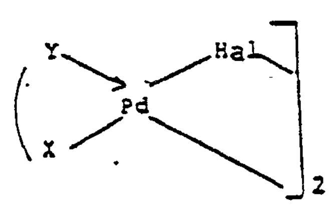

wherein Hal represents a halogen, with a silver salt in the presence of one or more weakly coordinating ligands.

10. The process according to claim 7, characterized in that a complex is used, which has the following formula:

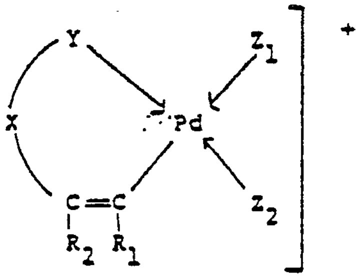

A⁻ ( IIc )

so as to obtain a heterocyclic complex of the formula

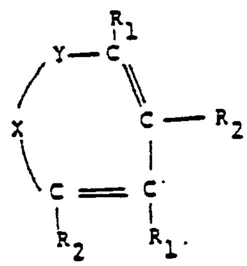

( VI )

**11.** The process according to claim 10, characterized in that the complex of formula (IIc) is obtained from a complex having the formula:

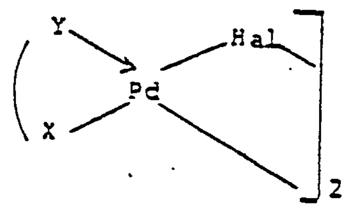

( V )

by reacting it with an alkyne of the formula

$R_1 - C \equiv C - R_2$ (III)

leading to a complex having the formula:

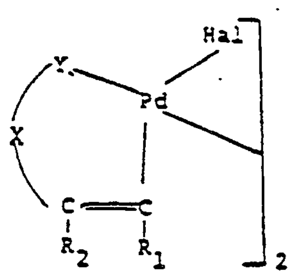

( VII )

and that this complex is converted into a complex of the formula IIc.

**12.** A sulphur containing heterocyclic compound, having the formula:

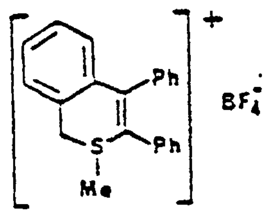

**13.** A heterocyclic compound having the formula

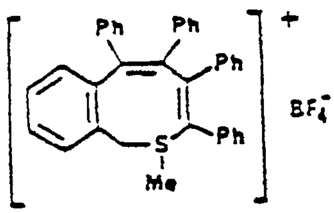

**14.** An intermediate product having the formula:

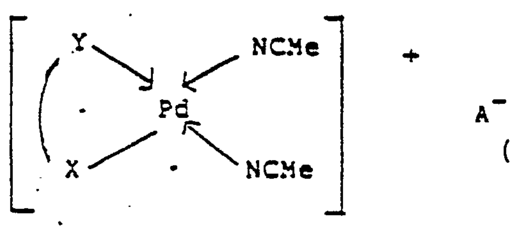

(IIb)

wherein -X-Y- represents an organic residue linked to the double bond at Y via a positively charged sulphur atom, and at X via a carbon atom; and A represents an anion.

**15.** The compound according to claim 14, characterized in that it has the following formula:

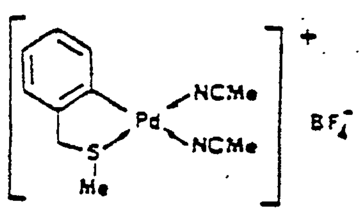

**16.** Halogen containing complexes having the formula

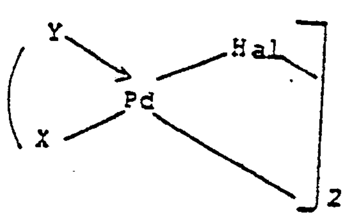

(V)

wherein Hal represents a halogen, and wherein -X-Y- represents an organic residue linked to the double bond at Y via a positively charged sulphur atom, and at X via a carbon atom.

**17.** The compounds according to claim 16, having the formula

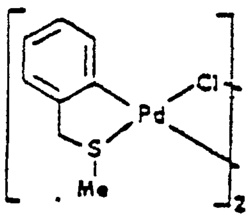

**18.** The cationic complex having the formula

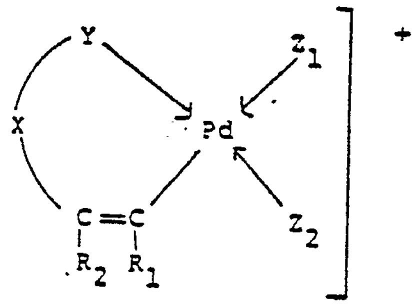

(IIc)

wherein -X-Y-, $R_1$, $R_2$, $Z_1$ and $z_2$ have the meaning given in claim 7.

**19.** Halogen containing compound having the formula:

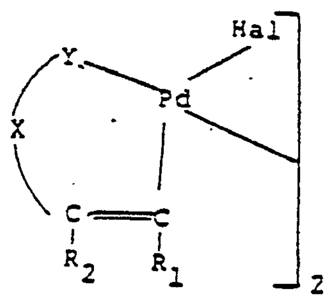

(VII)

wherein -X-Y-, $R_1$, $R_2$, have the meaning given in claim 7.

**20.** The compounds according to claim 19, having the formula

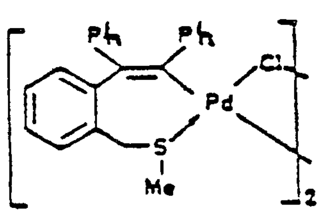

**Patentansprüche**

1. Verfahren zur Synthese von Heterozyklen aus Cyclo-Palladium-Komplexen, durch Reaktion mit Alkinen, dadurch gekennzeichnet, daß Cyclo-Palladium-Komplexe in Form eines jodhaltigen Komplexes der Formel

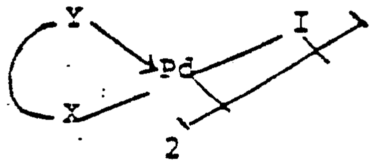

(I)

oder in Form eines kationischen Komplexes der Formel

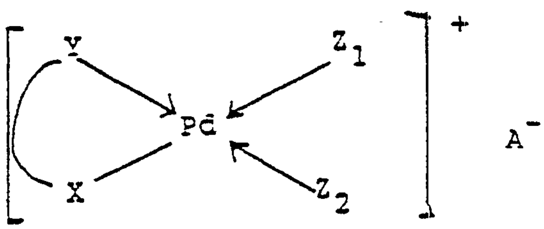

(II)

verwendet werden, worin Y - X einen organischen Rest darstellt, der auf der Y-Seite über ein Stickstoff- oder Schwefelatom und auf der X-Seite über ein Kohlenstoffatom gebunden ist, $Z_1$ und $Z_2$ zwei schwach koordinierte Liganden darstellen, wie beispielsweise Nitrile-RCN, Äther-R-O-R', Thioäther-R-S'-R', Ketone-R-CO-R', Alkohole-ROH, THF, DMSO und DMF (in den genannten Formeln kann es sich bei R und R' um $C_1$-$C_6$-Alkylgruppen oder Arylgruppen handeln), und A repräsentiert ein Anion.

2. Verfahren nach Anspruch 1, dadurch gekennzeichnet, daß Y-X ein Rest ist, der von 8-Methylchinolin, N,N-Dimethylnaphtylamin, Benzolpyridin und N,N-Dimethylbenzolamin abgeleitet ist.

3. Verfahren nach Anspruch 1 oder 2, dadurch gekennzeichnet, daß man ein Alkin der Formel verwendet,

$R^1$ -C ≡ C - $R^2$ (III)

worin $R^1$ und $R^2$ unabhängig voneinander ausgewählt werden aus Wasserstoff, Arylgruppen wie Phenyl, $C_1$-$C_6$-Alkylgruppen, insbesondere, Halogen-$C_1$-$C_6$-alkylgruppen; $C_2$-$C_6$-Alkenylgruppen; Gruppen der Formel $-COOR_3$ oder $-CO-R_3$, worin $R_3$ für eine $C_1$-$C_6$ -Alkylgruppe oder Arylgruppe steht; Gruppen der Formel $-CR_4R_5OH$, worin $R_4$ und $R_5$ ausgewählt werden aus Wasserstoff, $C_1$-$C_6$-Alkylgruppen und Arylgruppen, einer -CHO-Gruppe; Gruppen der Formel $-NR_6R_7$, worin $R_6$ und $R_7$ ausgewählt werden aus Wasserstoff und $C_1$-$C_6$-Alkylgruppen, Gruppen der Formel $-SR_8$ oder $-SO_2R_8$, worin $R_8$ ausgewählt wird aus $C_1$-$C_6$-Alkylgruppen und Arylgruppen, einer Trimethylsilylgruppe sowie solchen Gruppen, die durch eine $C_1$-$C_6$-Alkylenkette an die Dreifachbindung gebunden sind.

4. Verfahren nach einem der Ansprüche 1 bis 3, dadurch gekennzeichnet, daß die Reaktion durch Erhitzen in einem inerten Lösungsmittel durchgeführt wird.

5. Verfahren nach Anspruch 3, dadurch gekennzeichnet, daß man einen jodhaltigen Komplex der Formel

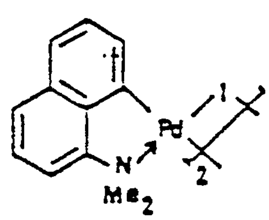

mit einem Alkin der Formel

$R^1 - C \equiv C - R^2$

reagieren läßt, worin $R^1$ und $R^2$ die in Anspruch 3 angegebene Bedeutung haben, unter Bildung einer heterozyklischen Verbindung der Formel

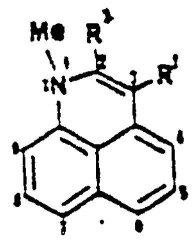

worin R1 und $R^2$ die zuvor angegebene Bedeutung besitzen.

6. Verfahren nach Anspruch 3, dadurch gekennzeichnet, daß man einen kationischen Komplex der Formel

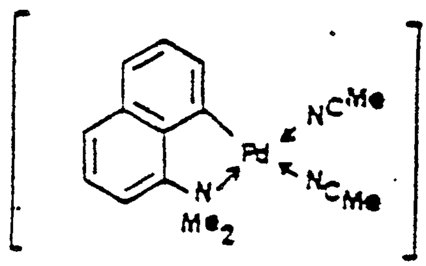 $BF_4$

mit einem Alkin der Formel

$R^1 - C = C - R^2$

reagieren läßt, worin $R^1$ und $R^2$ die in Anspruch 3 angegebene Bedeutung haben, unter Bildung einer heterozyklischen Verbindung der Formel

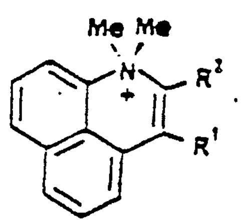 , $BF_4^-$

worin $R^1$ und $R^2$ die zuvor angegebene Bedeutung besitzen.

7. Verfahren zur Synthese schwefelhaltiger Heterozyklen der Formel

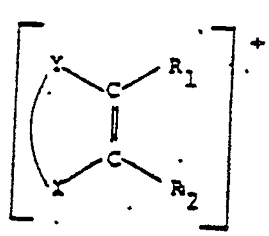 $A^-$ (IV)

worin -X-Y- einen organischen Rest darstellt, der auf der Y-Seite über ein positiv geladenes Schwefelatom und auf der X-Seite über ein Kohlenstoffatom an die Doppelbindung gebunden ist, worin $R^1$ und $R^2$ unabhängig voneinander jeweils ausgewählt werden aus Wasserstoff, Arylgruppen wie Phenyl, Alkylgruppen, insbesondere $C_1$-$C_6$-Alkylgruppen, Halogen-$C_1$-$C_6$-alkylgruppen; $C_2$-$C_6$-Alkenylgruppen; Gruppen der Formel -$COOR_3$ oder -CO-$R_3$, worin $R_3$ für eine $C_1$-$C_6$-Alkylgruppe oder Arylgruppe steht; Gruppen der Formel -$CR_4R_5OH$, worin $R_4$ und $R_5$ ausgewählt werden aus Wasserstoff, $C_1$-$C_6$-Alkylgruppen und Arylgruppen, einer -CHO-Gruppe, Gruppen der Formel -$NR_6R_7$, worin $R_6$ und $R_7$ ausgewählt werden aus Wasserstoff und $C_1$-$C_6$-Alkylgruppen, Gruppen der Formel -$SR_8$ oder -$SO_2R_8$, worin $R_8$ ausgewählt wird aus $C_1$-$C_6$-Alkylgruppen und Arylgruppen, einer Trimethylsilylgruppe sowie solchen Gruppen, die über eine $C_1$-$C_6$-Alkylenkette an die Dreifachbindung gebunden sind; und A ein Anion darstellt, nach einem der Ansprüche 1 bis 4, dadurch gekennzeichnet, daß man einen kationischen Komplex der Formel

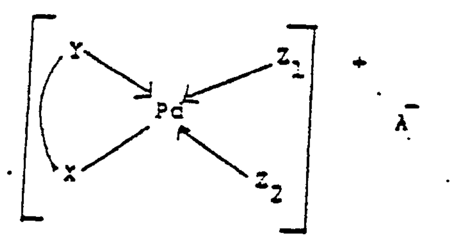

(IIa)

reagieren läßt, worin -X-Y- und A die oben angegebene Bedeutung haben und $Z_1$ und $Z_2$ zwei schwach koordinierende Liganden darstellen, mit einem Alkin der Formel

$$R_1 - C \equiv C - R_2 \quad \text{(III)}$$

reagieren läßt.

8. Verfahren nach Anspruch 7, dadurch gekennzeichnet, daß man einen kationischen Komplex folgender Formel verwendet

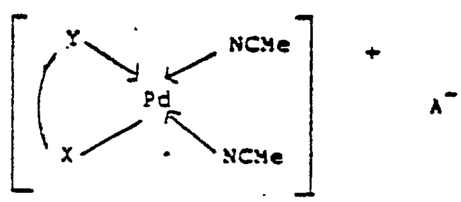

(IIb)

9. Verfahren nach Anspruch 7, dadurch gekennzeichnet, daß man die kationischen Komplexe der Formel (IIa) durch Umsetzung eines Komplexes der Formel

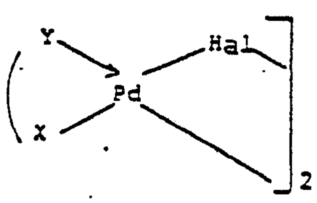

worin Hal ein Halogen darstellt, mit einem Silbersalz in Gegenwart von einem oder mehrerer schwach koordinierender Liganden herstellt.

10. Verfahren nach Anspruch 7, dadurch gekennzeichnet, daß man einen Komplex der Formel

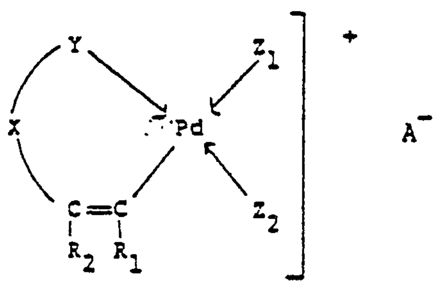

(IIc)

verwendet und eine heterozyklische Verbindung der Formel erhält

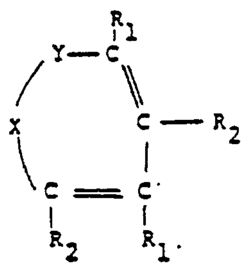

(VI)

**11.** Verfahren nach Anspruch 10, dadurch gekennzeichnet, daß man einen Komplex der Formel (IIc) aus einem Komplex der Formel

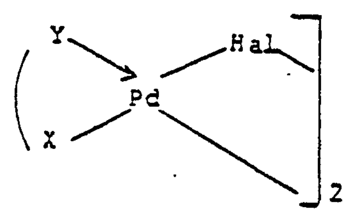

(V)

herstellt durch Umsetzung mit einem Alkin der Formel

$R_1 - C \equiv C - R_2$ (III)

unter Bildung eines Komplexes der Formel

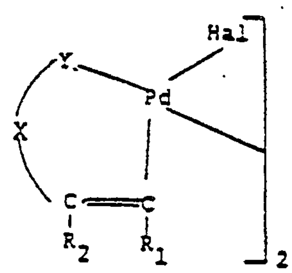

(VII)

und daß man diesen Komplex in einen Komplex der Formel IIc überführt.

**12.** Schwefelhaltige heterozyklische Verbindung der Formel

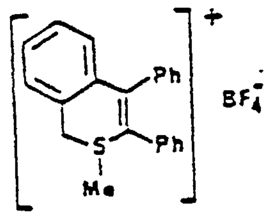

**13.** Heterozyklische Verbindung der Formel

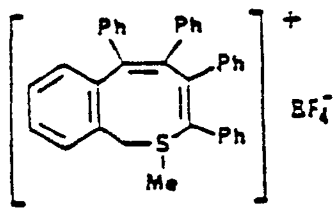

**14.** Zwischenprodukt der Formel

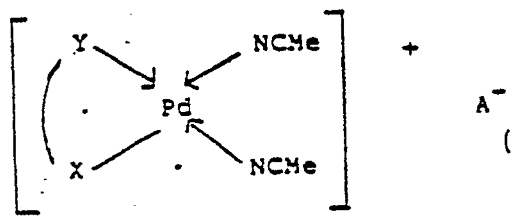

(IIb)

worin -X-Y- einen organischen Rest darstellt, der auf der Y-Seite über ein Schwefelatom und auf der X-Seite über ein Kohlenstoffatom an die Doppelbindung gebunden ist; und worin A ein Anion ist.

**15.** Verbindung nach Anspruch 14, der Formel

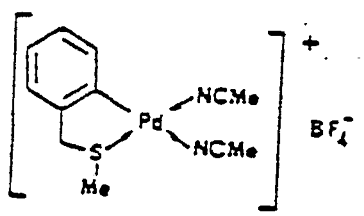

**16.** Halogenhaltige Komplexe der Formel

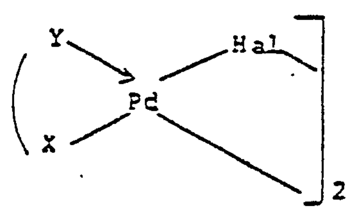

(V)

worin Hal ein Halogenatom und -X-Y- einen organischen Rest darstellen, der auf der Y-Seite über ein positiv geladenes Schwefelatom und auf der X-Seite über ein Kohlenstoffatom an die Doppelbindung gebunden ist.

**17.** Verbindungen nach Anspruch 16, der Formel

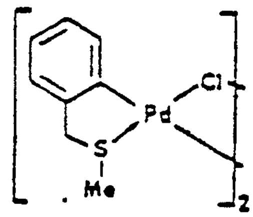

**18.** Kationische Komplexe der Formel

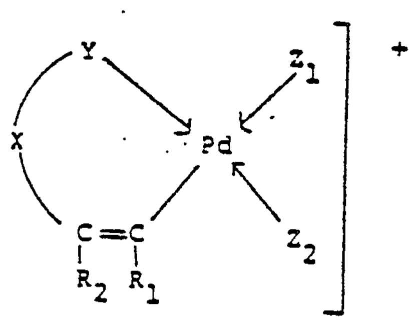

(IIc)

worin -X-Y-, $R_1$, $R_2$, $Z_1$ und $Z_2$ die in Anspruch 7 angegebene Bedeutung haben.

**19.** Halogenhaltige Verbindungen der Formel

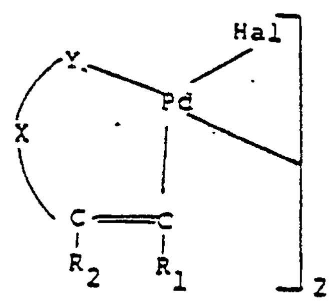

(VII)

worin -X-Y-, $R_1$, und $R_2$, die in Anspruch 7 angegebene Bedeutung haben.

**20.** Verbindungen nach Anspruch 19, der Formel